# EUROPÄISCHE PATENTSCHRIFT

(11) **EP 4 017 562 B1**
(45) Veröffentlichungstag und Bekanntmachung des Hinweises auf die Patenterteilung: **04.10.2023**
(21) Anmeldenummer: 20757231.4
(22) Anmeldetag: 04.08.2020
(51) Int. Cl.: A61M 15/00

(54) **SPENDER**
DISPENSER
DISTRIBUTEUR

(30) Priorität: 19.08.2019 DE 202019104547 U
(43) Veröffentlichungstag der Anmeldung: 29.06.2022
(73) Patentinhaber: RPC Formatec GmbH, 97638 Mellrichstadt (DE)
(72) Erfinder: THAU, Markus, 97638 Mellrichstadt (DE); KRIEGER, Johannes, 97638 Mellrichstadt (DE)
(74) Vertreter: Müller, Enno
(86) Internationale Anmeldenummer: PCT/EP2020/071908
(87) Internationale Veröffentlichungsnummer: WO 2021/032469

(56) Entgegenhaltungen:
- DE-A1-102008 023 376

## Beschreibung

### Gebiet der Technik

Die Erfindung betrifft einen Spender für in einer gesonderten Verpackung enthaltene, pulverförmige Massen, insbesondere in einer Blisterverpackung enthaltene Massen, wobei die Verpackung ein Behälterteil mit abziehbarer Behälterabdeckung aufweist, wobei weiter ein eingesaugter Luftstrom nach Entfernen der Behälterabdeckung vom Behälterteil zum überwiegenden Teil durch das Behälterteil gesaugt wird, derart, dass zwei durch Schlitze eines um eine Schwenkachse schwenkbaren Deckels eintretende Luftströme das Behälterteil von seinen beiden Enden her entleeren und nach Vereinigung in eine, einem Mundstück vorgeschalteten Wirbelkammer gelangen, wobei darüber hinaus in einer radialen Erstreckung zu der Schwenkachse in einem von dem Deckel abgedeckten Gehäuseteil Außenluft- Eintrittsöffnungen für einen dritten Luftstrom ausgebildet sind.

### Stand der Technik

Ein Spender der in Rede stehenden Art ist beispielsweise aus der WO 2009/138344 A1 bekannt.

Weiter ist zum Stand der Technik auf die DE 10 2008 023 376 A1 zu verweisen.

Bei dem aus letztgenannter Druckschrift bekannten Spender sind die Außenluft-Eintrittsöffnungen in dem Deckel selbst, durch rippenartigen Stege unterbrochen, ausgebildet. Der Nutzer greift hierauf bei der Benutzung. Der Deckel ist nahezu konturgleich mit dem darunter liegenden Gehäuse ausgebildet.

### Zusammenfassung der Erfindung

Ausgehend von dem dargelegten Stand der Technik beschäftigt sich die Erfindung mit der Aufgabenstellung, einen Spender der genannten Art handhabungstechnisch vorteilhaft auszubilden.

Diese Aufgabe ist beim Gegenstand des Anspruches 1 gelöst, wobei darauf abgestellt ist, dass der Deckel in einem die Außenluft-Eintrittsöffnungen für den dritten Luftstrom zugeordneten Teilbereich über eine Breite, welche einer Hälfte oder weniger der Breite des Deckels entspricht, mit einer bezüglich der Schwenkachse radialen Verlängerung ausgebildet ist, wobei die Verlängerung zugleich einen Untergreifvorsprung zum Öffnen des Deckels bildet und über einen benachbarten, nicht verlängerten Deckelabschnitt hinweg frei auskragt und das Ausmaß der Verlängerung im Hinblick auf den nicht verlängerten Deckelabschnitt einem Fünftel oder mehr der Breite des Deckels entspricht.

Zufolge dieser Ausgestaltung ist ein Spender der in Rede stehenden Art in gebrauchsvorteilhafter Weise weiter verbessert. Die vorgesehene, exponiert ausgebildete Verlängerung des Deckels bietet eine günstig zu ergreifende Handhabe zur Betätigung des Deckels, insbesondere zum Verschwenken des Deckels um dessen Schwenkachse. Die Handhabung des Spenders, insbesondere zum Öffnen des Deckels, ist zufolge der exponierten Ausbildung der Verlängerung intuitiv.

Darüber hinaus überfängt die deckelseitige Verlängerung die in dem Gehäuseteil vorgesehenen Außenluft-Eintrittsöffnungen für den dritten Luftstrom in der Deckelverschlussstellung vordachartig. Die Verlängerung kragt mit Blick auf eine Seitenansicht gegen den Spender, in welcher Seitenansicht sich die geometrische Schwenkachse als Punkt darstellt, ausgehend von einem Wurzelbereich der Verlängerung, in welchem diese in den sich über die gesamte Breite erstreckenden Deckelabschnitt einläuft, über den die Gesamtbreite weisenden Deckelabschnitt hinaus.

Dabei kann das Ausmaß der Verlängerung, beispielsweise ausgehend von dem vorbeschriebenen Wurzelbereich, und somit im Hinblick auf den benachbarten, nicht verlängerten Bereich, einem Viertel oder mehr der Breite, insbesondere der größten Breite des Deckels, bevorzugt gemessen parallel zur Schwenkachse betrachteten Breite des Deckels entsprechen.

Die Verlängerung kann, wie auch bevorzugt, einstückig und/ oder materialeinheitlich mit dem Deckel ausgebildet sein. So kann weiter bei einer bevorzugten Ausgestaltung des Deckels als Kunststoff-Spritzteil die Verlängerung zugleich in diesem Spritzverfahren zur Herstellung des Deckels ausgebildet werden, gegebenenfalls in einem Zwei-Komponenten-Spritzverfahren, bei welchem insbesondere bezüglich der Verlängerung eine zweite Kunststoffkomponente vorgesehen sein kann. Die Verlängerung kann dabei vollständig oder auch nur partiell, beispielsweise rand- oder oberflächen- beziehungsweise unterflächenseitig mit einer zweiten Kunststoff-Komponente versehen beziehungsweise durch diese ausgebildet sein.

Die vor- und nachstehend angegebenen Bereiche beziehungsweise Wertebereiche oder Mehrfachbereiche schließen hinsichtlich der Offenbarung auch sämtliche Zwischenwerte ein, insbesondere in 1/10-Schritten der jeweiligen Dimension, gegebenenfalls also auch dimensionslos. Beispielsweise beinhaltet die Angabe eine Hälfte (0,5-Fach) oder weniger auch die Offenbarung von 0,49 oder weniger, die Offenbarung von einem Viertel (0,25-Fach) oder mehr auch die Offenbarung von beispielsweise 0,26 oder mehr. Diese Offenbarung kann einerseits zur Eingrenzung einer genannten Bereichsgrenze von unten und/oder oben, alternativ oder ergänzend aber zur Offenbarung eines oder mehrerer singulärer Werte aus einem jeweilig angegebenen Bereich dienen.

### Kurze Beschreibung der Zeichnungen

Nachstehend ist die Erfindung anhand der beigefügten Zeichnungen erläutert, die aber lediglich ein Ausführungsbeispiel darstellt. Die Zeichnung zeigt:
- Fig. 1: einen Spender der in Rede stehenden Art in einer perspektivischen Darstellung, die Nichtbenutzungsstellung betreffend;
- Fig. 2: den Spender in Seitenansicht gemäß Pfeil II in Figur 1;
- Fig. 3: den Spender in einer weiteren perspektivischen Darstellung;
- Fig. 4: eine weitere perspektivische Darstellung des Spenders;
- Fig. 5: den Spender in Draufsicht;
- Fig. 6: den Schnitt gemäß der Linie VI-VI in Figur 5;
- Fig. 7: den Schnitt gemäß der Linie VII-VII in Figur 5;
- Fig. 8: den Spender in einer weiteren perspektivischen Darstellung, nach Abnahme einer Saugmund-Schutzkappe;
- Fig. 9: die Draufsicht hierzu;
- Fig. 10: den Spender in perspektivischer Darstellung, eine Bereitschaftsstellung zur Bestückung mit einem Behälterteil betreffend;
- Fig. 11: eine weitere perspektivische Darstellung des Spenders in der Bestückungs-Bereitschaftsstellung, bei in explosionsperspektivischer Anordnung eines Zwischenbodens;
- Fig. 12: den Spender in explosionsperspektivischer Darstellung;
- Fig. 13: eine weitere explosionsperspektivische Darstellung des Spenders;
- Fig. 14: eine weitere perspektivische Darstellung des Spenders in der Bestückungs-Bereitschaftsstellung im Zuge der Bestückung mit einem Behälterteil;
- Fig. 15: den Schnitt gemäß der Linie XV - XV in Figur 9 bei eingesetztem Behälterteil;
- Fig. 16: eine der Figur 15 entsprechende Längsschnittdarstellung, jedoch die Spender-Bereitschaftsstellung betreffend;
- Fig. 17: eine der Figur 9 entsprechende Draufsicht, jedoch die Stellung gemäß Figur 16 betreffend;
- Fig. 18: den Schnitt gemäß der Linie XVIII - XVIII in Figur 16;
- Fig. 19: den Schnitt gemäß der Linie XIX-XIX in Figur 16;
- Fig. 20: eine der Figur 16 entsprechende Darstellung, jedoch im Zuge eines Inhaliervorganges;
- Fig. 21: den Schnitt gemäß dem Schnittverlauf XXI-XXI in Figur 20;

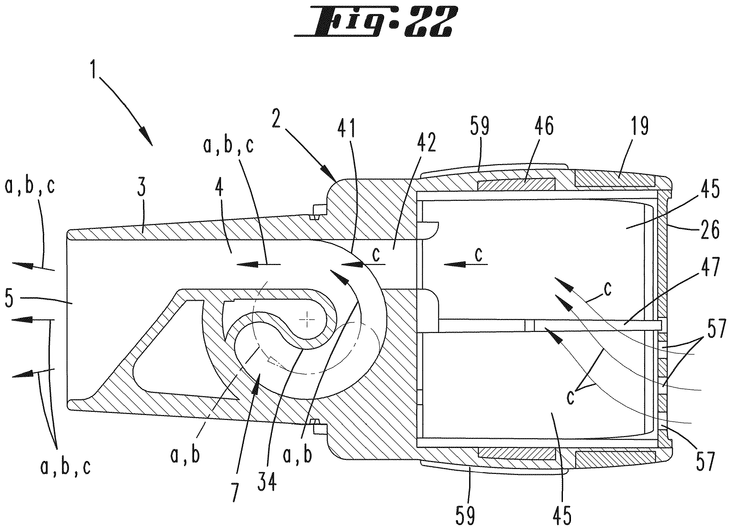

- Fig. 22: den Schnitt gemäß dem Schnittverlauf XXII-XXII in Figur 20.

### Beschreibung der Ausführungsformen

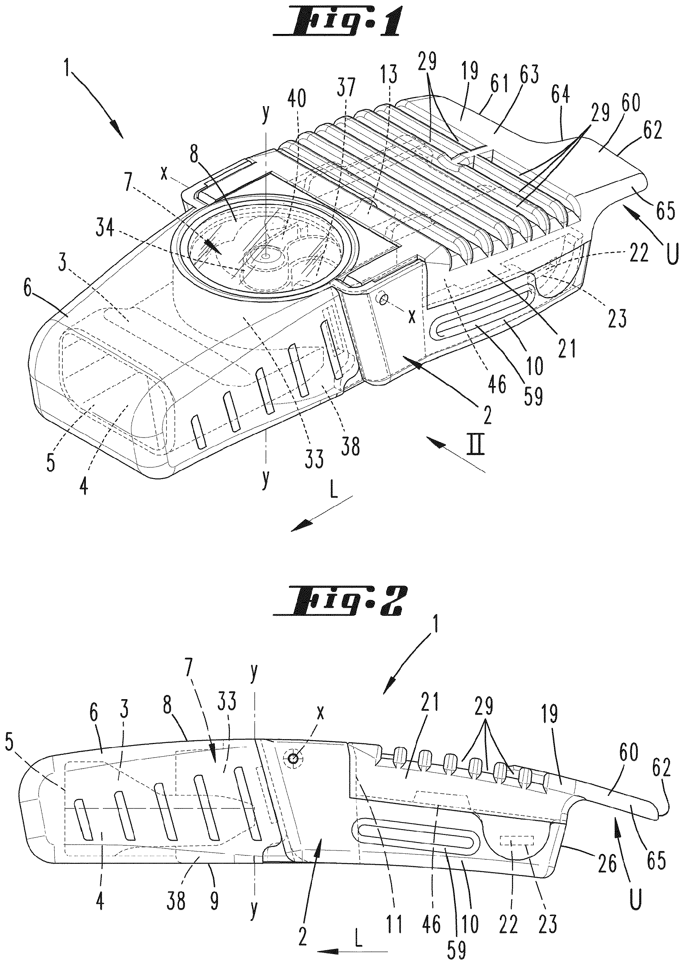

Dargestellt und beschrieben ist zunächst mit Bezug zu den Figuren 1 und 2 ein Spender 1 in Art eines Inhalators, welcher als bequem mitführbares Taschengerät realisiert ist. Dieses kann ein im Wesentlichen langgestreckt rechteckiges Gehäuse 2 aufweisen, mit einem Längen-Breitenverhältnis von etwa 2:1 bis 2,5:1 und einer senkrecht zur Längserstreckung betrachteten Höhe, die etwa einem Viertel des Längserstreckungsmaßes entspricht. Die Teile des Spenders 1 sind bevorzugt als Kunststoff-Spritzteile realisiert.

In Verlängerung der Längserstreckung L des Gehäuses 2 ragt von diesem ein Mundstück 3 ab. Der Übergang von Gehäuse 2 zum Mundstück 3 kann bei insgesamt einteiliger Ausgestaltung mit Bezug auf die Breitenerstreckung des Gehäuses 2 tailliert sein.

Das Mundstück 3 ist in Längserstreckung L durchsetzt von einem Saugkanal 4, der ausgangsseitig in einem Saugmund 5 endet.

Bei Nichtbenutzung des Spenders 1 ist das Mundstück 3 gemäß den Darstellungen in den Figuren 1 und 2 bevorzugt durch eine Verschlusskappe 6 überdeckbar. Diese ist, die Breite des Gehäuses 2 im Wesentlichen aufnehmend, im Bereich des taillenartigen Übergangs von Mundstück 3 zum Gehäuse 2 steckhalterbar.

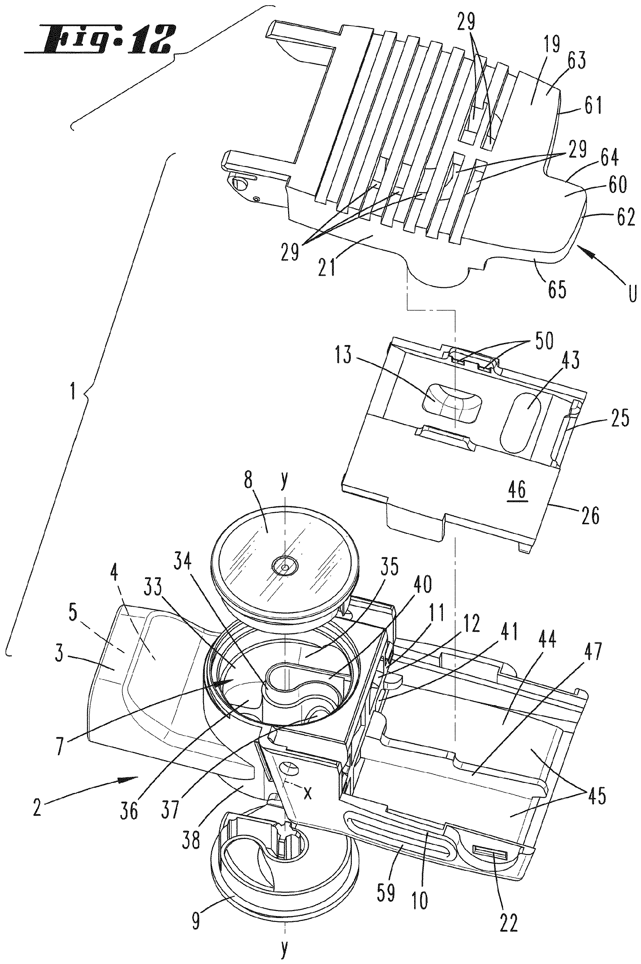

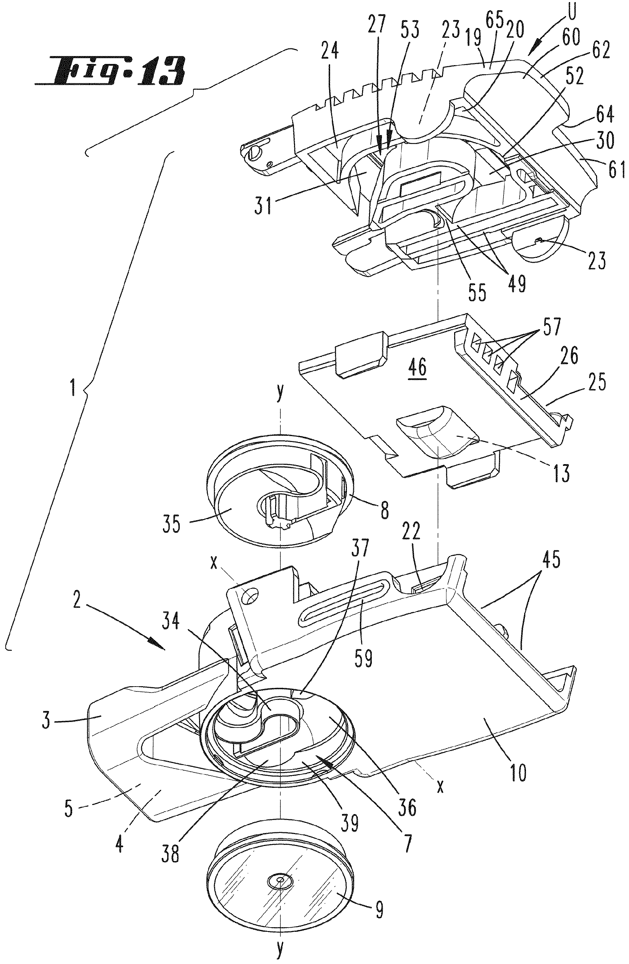

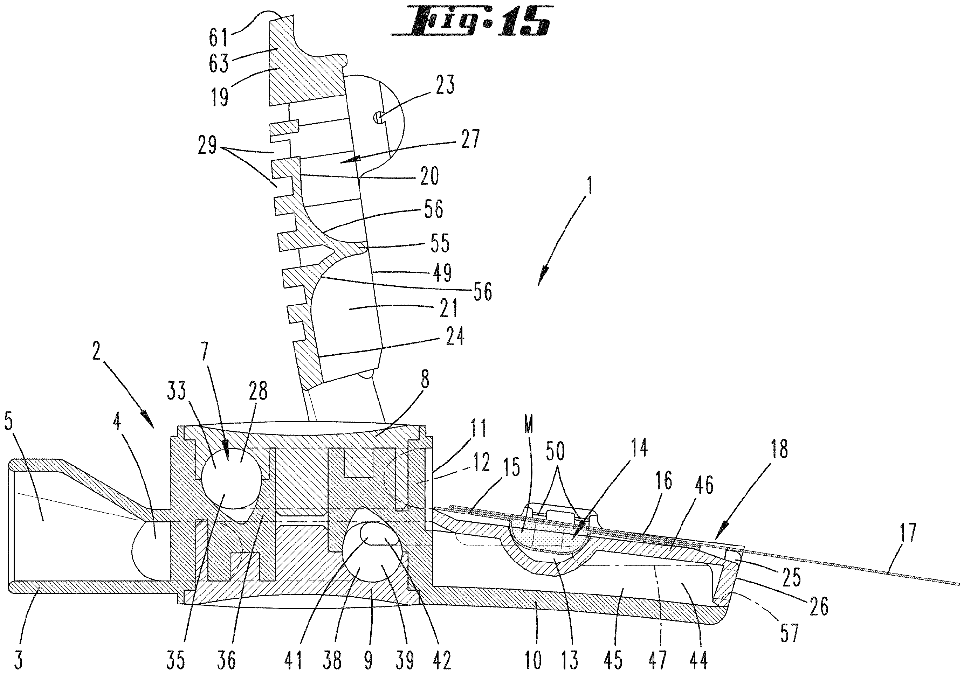

Dem Übergangsbereich von Gehäuse 2 zum Mundstück 3 zugeordnet ist in dem Gehäuse 2 eine kreisrunde Wirbelkammer 7 mit einer im Wesentlichen senkrecht zum Saugkanal 4 verlaufenden Wirbelkammerachse y ausgebildet (vergleiche Figur 15). Diese Wirbelkammer 7 erstreckt sich über zwei Stockwerke im Wesentlichen über die gesamte Höhe des Gehäuses 2, wobei die Kammerdecke 8 sowie der Kammerboden 9 durch kreisscheibenförmige Deckplatten gebildet sind, die bevorzugt an dem Gehäuse 2 steckgehaltert sind (vergleiche Figuren 12 und 13). Kammerdecke 8 und Kammerboden 9 können transparent gestaltet sein. Möglich ist auch eine opake Ausgestaltung.

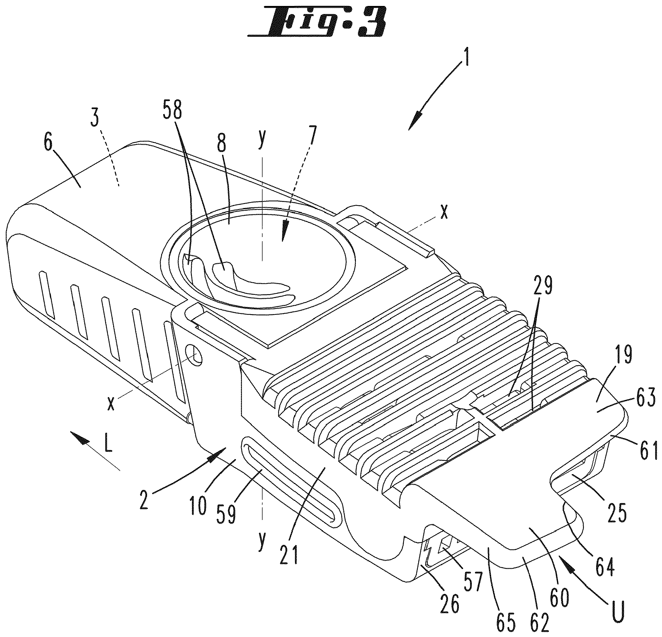

Darüber hinaus können gemäß einer möglichen Ausführungsform Kammerdecke 8 und Kammerdecke 9, wie beispielsweise aus Figur 3 ersichtlich, mit schlitzartigen, beispielsweise im Wesentlichen konzentrisch zu der Wirbelkammerachse y verlaufenden Ausnehmungen 58 versehen sein. Diese können nach axial außen offen ausgebildet sein. In Richtung auf die überdeckte Wirbelkammer 7 hingegen sind diese geschlossen.

Die Ausnehmungen 58 können beispielsweise als Montagehilfe dienen. Darüber hinaus werden durch diese Ausbildung vergleichsweise dünne Kammerwandungen in der Wirbelkammer 7 erreicht und somit die Gefahr von Einfallstellen reduziert.

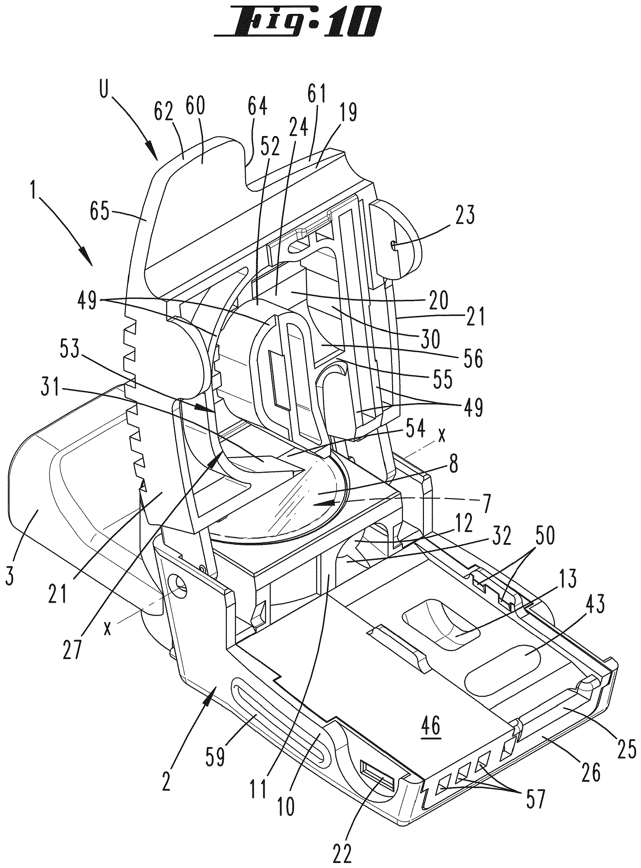

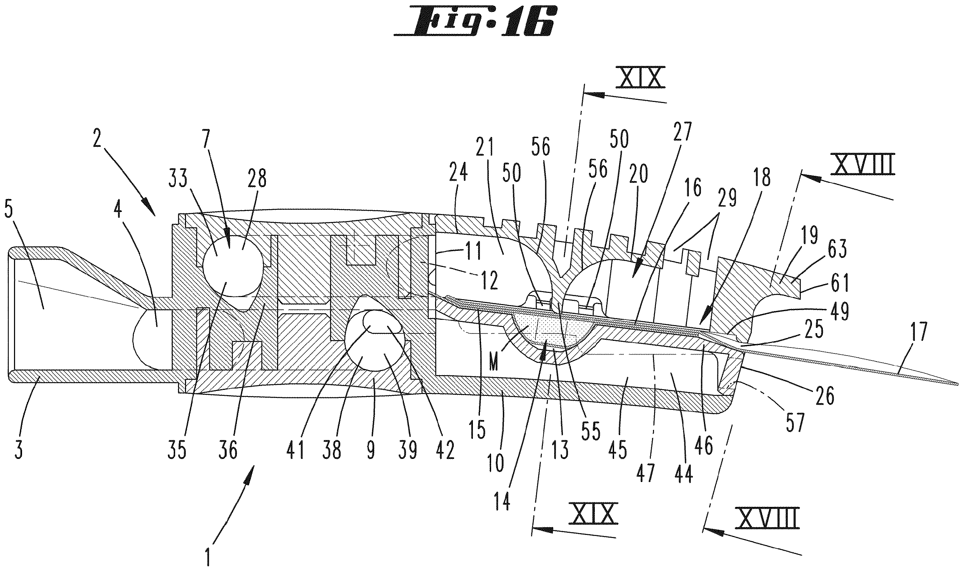

Das dem Mundstück 3 abgewandte, sich an der Wirbelkammer 7 anschließende Gehäuseteil 10 kann höhenmäßig abgestuft ausgebildet sein. Die verbleibende, gegenüber der Wirbelkammer-Decke 8 vertikal versetzte Oberflächenebene dieses Gehäuseteiles 10 erstreckt sich etwa mit Bezug auf die Höhenerstreckung des Gehäuses 2 in einer Mittenebene (vergleiche beispielsweise Figuren 10, 15 und 16). Stirnseitig, das heißt der abgesetzten Ebene des Gehäuseteiles 10 zugewandt, mündet in der sich an der Wirbelkammer 7 gegenüberliegend zum Mundstück 3 anschließenden Stufe 11 ein im Wesentlichen in Längserstreckung des Gehäuses 2 ausgerichteter Luftkanal 12. Dieser weist einen Durchmesser auf, der bevorzugt etwa dem 0,7- bis 0,8-Fachen der freien Stufenhöhe entspricht. Anderendig mündet dieser Luftkanal 12 in der Wirbelkammer 7, insbesondere in dem oberen Stockwerk der Wirbelkammer 7.

Der höhenmäßig abgestufte Bereich des Gehäuseteiles 10 ist zunächst nach oben, abgewandt zu einem Gehäuseteilboden, offen gestaltet, hierbei im Wesentlichen in Längserstreckung getrennt durch einen mit Bezug auf die quer zur Längserstreckung betrachtete Breite mittig angeordneten Vertikalsteg 47, woraus sich entsprechend zwei zumindest annähernd gleich große Bereiche beidseitig des Vertikalsteges 47 ergeben. Diese Bereiche sind überdeckt von einem Zwischenboden 46, der entsprechend die abgesetzte Oberflächenebene des Gehäuseteiles 10 ausbildet. Der Zwischenboden 46 schließt im wesentlichen umlaufend randseitig mit der Stufenunterkante, weiter unterhalb der Mündung des Luftkanals 12 und mit der Randbegrenzung des abgestuften Bereichs des Gehäuseteiles 10 ab und ist weiter bevorzugt mit dem Gehäuseteil 10 betrieblich nicht lösbar verrastet.

In dem Zwischenboden 46 ist eine zur ebenen Oberfläche hin sich öffnende Aufnahme 13 ausgebildet. Diese weist einen langlochartigen Grundriss auf und ist weiter mit Abstand zur Luftkanalmündung in der Stufe 11 in axialer Verlängerung des Luftkanales 12 ausgeformt. Die Kontur und Tiefe der wannenartigen Aufnahme 13 ist angepasst an die Kontur und Höhe eines aufzunehmenden Behälterteils 14, entsprechend in Längserstreckung jeweils endseitig zur Aufnahme eines kapselartigen Behälterteiles verrundet.

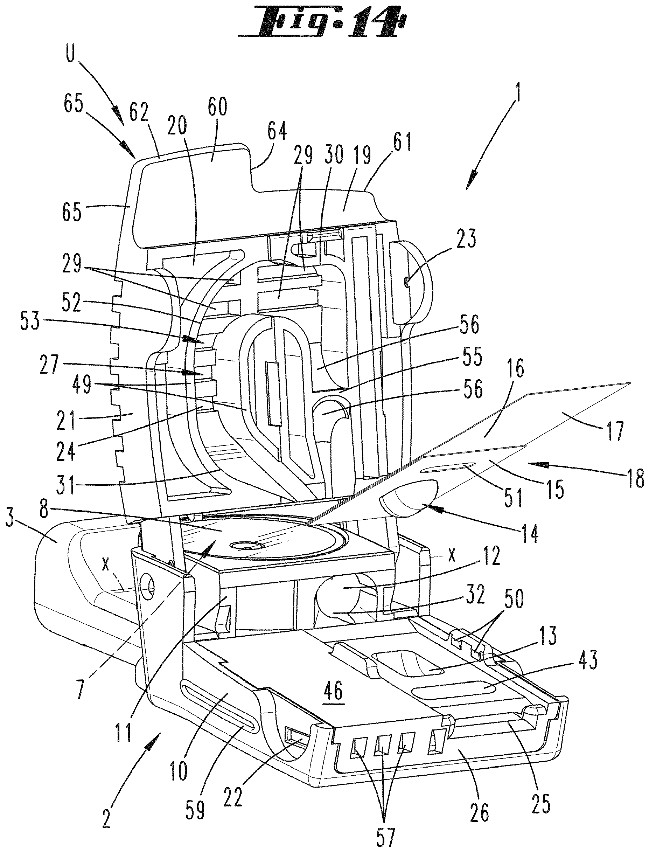

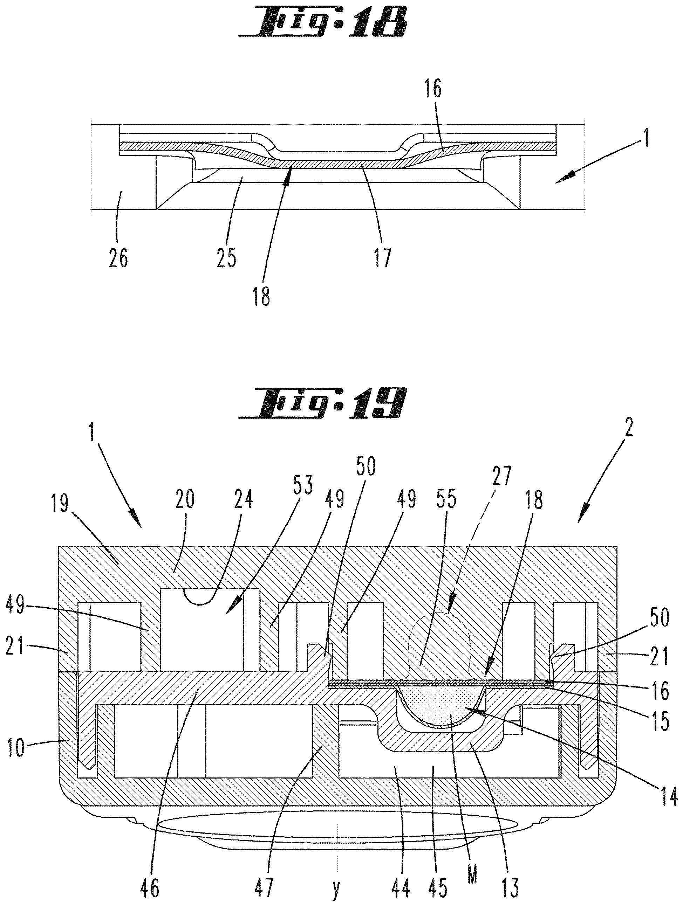

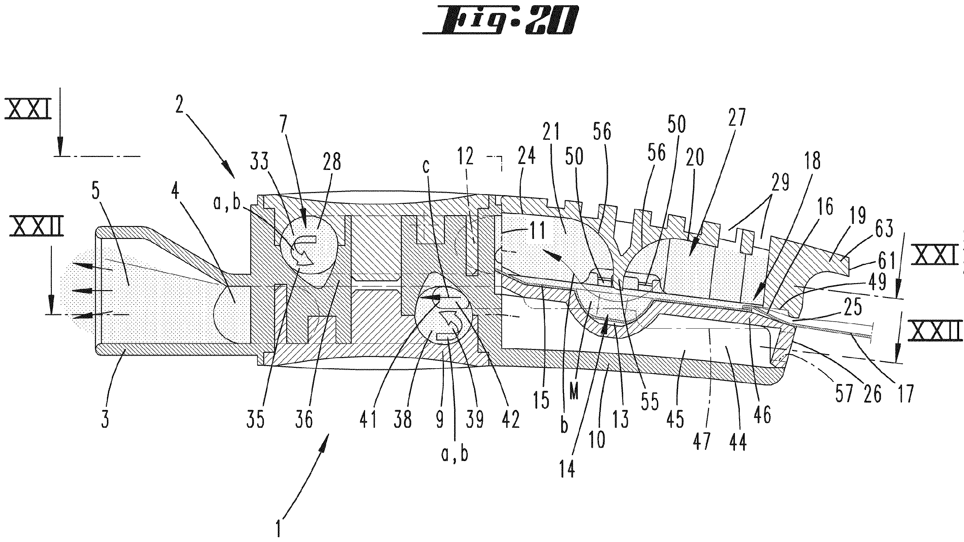

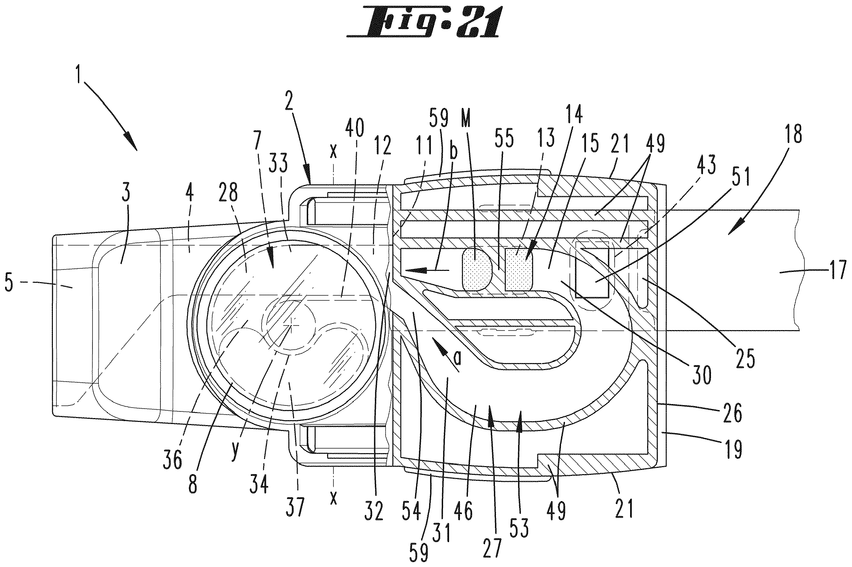

Wie aus den weiteren Darstellungen insbesondere der Figuren 14 bis 21 zu erkennen, ist das Behälterteil 14 in Art einer Blisterverpackung gestaltet, wobei das Behälterteil 14 eine pulverförmige Masse M bevorratet. Hierzu ist das Behälterteil 14 wannenartig aus einem Kunststoffmaterial gefertigt, wobei weiter sich an dem umlaufenden Öffnungsrand des Behälterteiles 14 ein ebenflächiger Stützabschnitt 15 anschließt. Insgesamt ist das Behälterteil 14 als Vertiefung aus dem Stützabschnitt 15 gebildet.

Die in dem Behälterteil 14 bevorratet Masse M ist in Nichtbenutzungsstellung versiegelt durch eine den Stützabschnitt 15 und das Behälterteil 14 vollflächig überdeckenden Aluminiumfolie, die die Behälterabdeckung 16 darstellt. Diese Behälterabdeckung 16 ist zur Freigabe des Behälterteils 14 bzw. der darin bevorrateten Masse M von dem Stützabschnitt 15 abziehbar, wozu die Aluminiumfolie bzw. die Behälterabdeckung 16 ausgehend von einem quer zur Längserstreckung des Behälterteils 14 ausgerichteten Schmalbereich des Stützabschnittes 15 sich weiter in Gegenrichtung frei erstreckt und hierbei den das Behälterteil 14 und den Stützabschnitt 15 versiegelnden Bereich der Behälterabdeckung 16 frei aufliegend überdeckt. Das freie Ende der Behälterabdeckung 16 ragt frei über die dem Wendebereich der Behälterabdeckung 16 abgewandten Stirnrandkante des Stützabschnittes 15 aus, zur Ausbildung einer fahnenartigen Abziehhandhabe 17.

Die in dem Zwischenboden 46 ausgeformte Aufnahme 13 für das Behälterteil 14 ist in einem gegenüber der Zwischenboden-Oberfläche vertikal tiefer versetzten Bereich positioniert. Der Vertikalversatz entspricht im Wesentlichen der Materialstärke des sich aus Behälterteil 14, Behälterabdeckung 16 und Stützabschnitt 15 zusammensetzenden Blisters 18 außerhalb des Behälterteils 14. Weiter ist insbesondere die in Längserstreckung des Gehäuses 2 betrachtete Länge der Vertiefung angepasst an die in Längserstreckung des Behälterteiles 14 betrachtete Länge des Stützabschnittes 15. Die die Aufnahme 13 umgebende Oberfläche der Vertiefung dient als Auflage des Stützabschnittes 15 bei eingelegtem Blister 18.

Dem Gehäuseteil 10 ist ein Deckel 19 zugeordnet. Dieser ist im Bereich der Stufe 11 um eine quer zur Längserstreckung des Gehäuses 2 gerichtete Schwenkachse x klappbar an dem Gehäuseteil 10 angelenkt. Der Deckel 19 weist eine Decke 20 auf. Beidseitig in Längserstreckung des Gehäuses 2 verlaufende Seitenwände 21 sind einstückig mit der Deckeldecke 20 ausgebildet und flankieren in der Deckelschließstellung die zugeordneten Seitenflächen des feststehenden Gehäuseteiles 10.

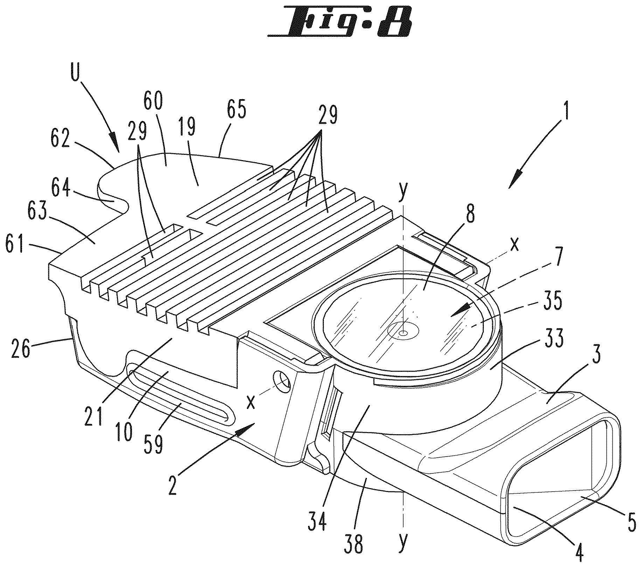

Wie beispielsweise auch in den Darstellungen der Figuren 3 bis 8 zu erkennen, kann der Deckel 19 mit einer in Längserstreckung L des Spenders 1 ausgerichteten Verlängerung 60 versehen sein. Diese kann zunächst und im Wesentlichen als Untergreifvorsprung U zur Schwenkbetätigung des Deckels 19 dienen.

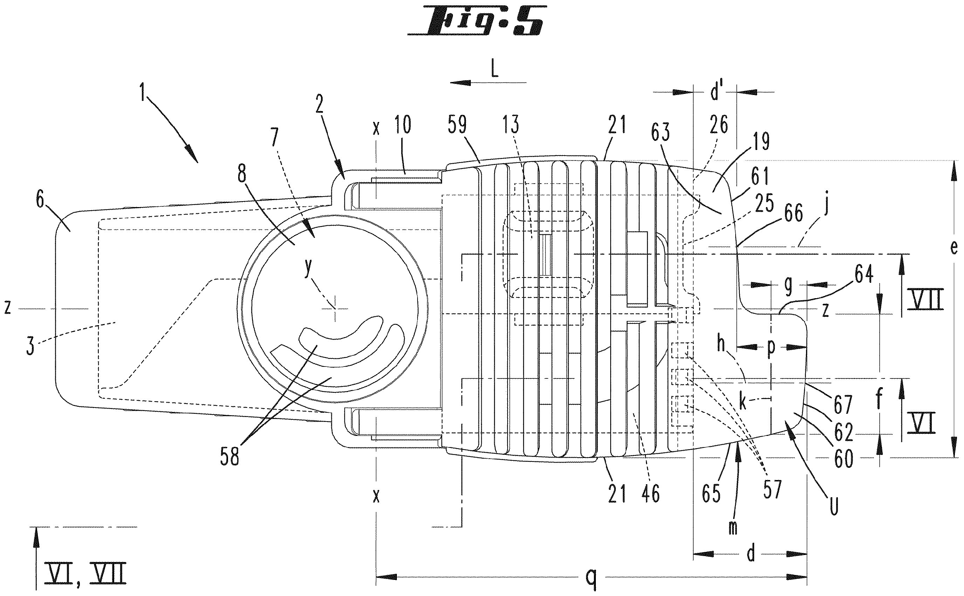

Die Verlängerung 60 kann sich hierbei in Bezug auf eine Draufsicht gemäß Figur 5 seitlich einer Längsmittelachse z des Spenders 1 über eine dem Mundstück 3 abgewandte Randfläche 61 des Deckels 19 hinaus erstrecken. Bevorzugt überschreitet die Verlängerung 60 in einer Breitenerstreckung, also in einer Richtung quer zur Längserstreckung L, nicht oder nicht wesentlich die Längsmittelachse z, gegebenenfalls diese berührend.

In Figur 5 ist eine Breite e des Deckels 19 abgetragen, gemessen an der breitesten Stelle des Deckels 19 quer zur Längserstreckung L beziehungsweise parallel zur Schwenkachse x. Diese Breite e des Deckels 19 kann etwa der Länge q, ausgehend von der Schwenkachse x, bis zur freien Stirnrandfläche 62 der Verlängerung 60, gemessen in Längserstreckung L, entsprechen, weiter etwa dem 1,1- bis etwa 2-Fachen dieser Länge q.

Entlang einer Hilfslinie k, die quer zur Längserstreckung L verlaufend mit Bezug auf eine Draufsicht gemäß Figur 5 die Verlängerung 60 durchsetzt, ergibt sich eine Breite f der Verlängerung 60, die bevorzugt kleiner ist als die Hälfte der Deckelbreite e, so gemäß dem dargestellten Ausführungsbeispiel etwa dem 0,35- bis 0,4-Fachen der Deckelbreite e entsprechend.

Die Hilfslinie k verläuft dabei mit einem Versatzmaß g zu dem in Längserstreckung L betrachteten äußersten Bereich der Stirnrandfläche 62, welches Versatzmaß g etwa dem 0,1-Fachen der Deckelbreite e entspricht.

Mit Bezug zu einer Rückwand 26 des Gehäuseteiles 10, in welcher Rückwand 26, wie weiter unten näher beschrieben, Außenluft-Eintrittsöffnungen 57 münden, ergibt sich eine in Längserstreckung L betrachtete Länge d der Verlängerung 60, die etwa dem 0,25- bis 0,4-Fachen, weiter beispielsweise etwa einem Drittel der Breite e und/oder der Deckellänge q entsprechen kann. Die Länge d ist hierbei entlang einer weiteren Hilfslinie h gemessen, welche Hilfslinie h in Längserstreckung L ausgerichtet die vorbeschriebene Hilfslinie k mittig durchsetzt.

Der benachbart zu der vorbeschriebenen Verlängerung 60 ausgebildete, die Randfläche 61 aufweisende Deckelabschnitt 63 ragt in einer bevorzugten Ausgestaltung ähnlich der Verlängerung 60 über die Rückwand 26 hinaus, dies jedoch nur mit einem vergleichsweise geringen Längenmaß d', das beispielsweise dem 0,25- bis 0,5-Fachen, weiter etwa einem Drittel des Längenmaßes d der Verlängerung 60 entsprechen kann. Das Längenmaß d' ist hierbei abgetragen entlang einer weiteren Hilfslinie j (vergleiche Figur 5), welche Hilfslinie j mittig der Breitenerstreckung des Deckelabschnittes 63 im Bereich der Rückwand 26, ausgehend von der Längsmittelachse z, in Längserstreckung L verläuft.

Wie weiter aus der Draufsichtdarstellung in Figur 5 zu erkennen, verläuft eine innere Seitenrandfläche 64 der Verlängerung 60 im Wesentlichen in paralleler Ausrichtung zu der Längsmittelachse z, wobei weiter bevorzugt jeweils im Übergang zu der Randfläche 61 und zu der Stirnrandfläche 62 der Verlängerung 60 ein Radius gegeben sein kann.

Die äußere Seitenrandfläche 65 verläuft bevorzugt stufenlos in die zugewandte Außenwandfläche des Deckels 19 ein, wobei die sich in der Draufsicht ergebende Randkante bevorzugt entlang eines Kreisbogens mit einem Radius m verläuft. Der Radius m kann etwa dem 1,1- bis 1,5-Fachen der Breite e des Deckels 19 entsprechen. Hieraus ergibt sich mit Bezug auf die Draufsicht gemäß Figur 5 eine Verjüngung der Verlängerung 60 hin zu dessen freien Ende.

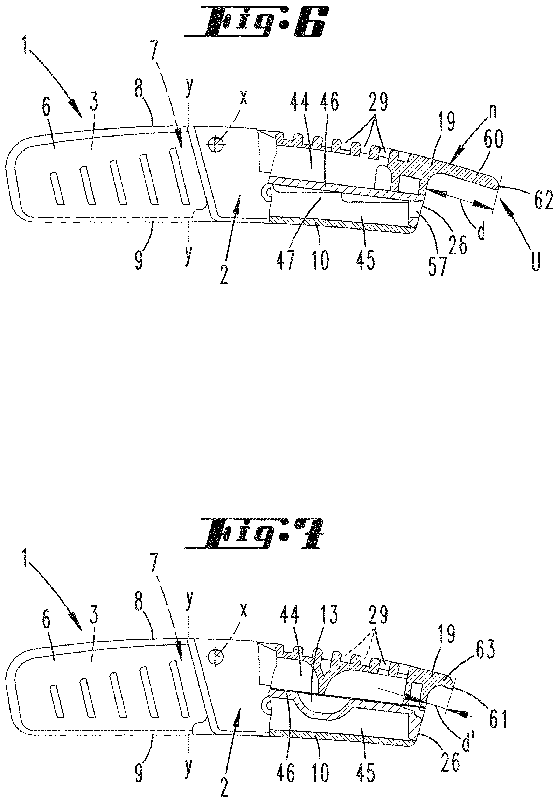

Insbesondere aus den Schnittdarstellungen in den Figuren 6 und 7 sind die unterschiedlichen freien Längen d und d' des Deckelabschnittes 63 und der Verlängerung 60 zu erkennen. Insbesondere diese Abschnitte (Deckelabschnitt 63 und Verlängerung 60) verlaufen in den Schnittdarstellungen bevorzugt entlang einer Kreislinie mit einem Radius n, wobei dieser Radius n bevorzugt einem Mehrfachen, beispielsweise einem 4- bis 6-Fachen, weiter bevorzugt etwa einem 5-Fachen der Deckelbreite e entsprechen kann. Das jeweilige freie Ende der Verlängerung 60 und des Deckelabschnittes 63 weist dabei schnabelartig tendenziell in Richtung auf eine Bodenebene des Spenders 1 beziehungsweise des Gehäuses 2.

Bevorzugt laufen der Deckelabschnitt 63 und die Verlängerung 60 insbesondere oberflächenseitig in einer gemeinsamen Ebene stufenlos in die übrige Deckelfläche ein.

Es ergibt sich weiter, insbesondere mit Bezug zu der Draufsicht in Figur 5, ein Ausmaß p, über welches die Verlängerung 60 über den benachbarten, nicht verlängerten Deckelabschnitt 63 hinweg frei ausragt. Dieses Ausmaß p ergibt sich durch ein Differenzmaß, gemessen in Längserstreckung L, zwischen einem Schnittpunkt 66 zwischen der mittig verlaufenden Hilfslinie j des Deckelabschnittes 63 und der Randfläche 61 sowie dem Schnittpunkt 67 zwischen der Hilfslinie h der Verlängerung 60 und dessen Stirnrandfläche 62. Erfindungsgemäß entspricht das Maß p dabei einem Fünftel oder mehr der Breite e des Deckels 19 weiter beispielsweise etwa dem 0,25- bis 0,3-Fachen der Breite e.

Die wie vorbeschrieben ausgebildete Verlängerung 60 des Deckels 19 bietet für den Nutzer einen handhabungsgünstigen, laschenartigen Untergreifvorsprung U, der ein günstiges Auf- und Zuschwenken des Deckels 19 ermöglicht.

Der hierdurch weiter bevorzugt vorgegebene Greifbereich zum Öffnen des Deckels 19 ist so zudem ausreichend beabstandet zu den Außenluft-Eintrittsöffnungen 57 in der Rückwand 26.

Der Deckel 19 ist in der Deckelverschlussstellung rastgehaltert, wozu an den Seitenwänden 21 des Deckels 19 Rastvorsprünge 23 vorgesehen sind, die in gehäuseteilseitige Rastvertiefungen 22 eintauchen. Die so gebildete Rasthalterung ist durch den Anwender zum Öffnen des Deckels 19 leicht überwindbar.

Zur günstigen Handhabung des Spenders 1, insbesondere im Zuge einer Deckelbetätigung, können wandungsaußenseitig der gehäuseteilseitigen Seitenwände, an denen auch die vorbeschriebenen Rastvertiefungen 22 ausgebildet sein können, rippenartig Vorsprungausbildungen 59 vorgesehen sein, die ein handhabungsgünstiges und sicheres Ergreifen ermöglichen.

Die Deckenunterseite 24 liegt in der Deckelverschlussstellung über deckenunterseitig angeformte Vertikalstege 49 auf der zugewandten Oberseite des Zwischenbodens 46 auf, so bevorzugt auf der die Aufnahme 13 aufnehmende Vertiefung umgebenden Planfläche. Bei eingelegtem Blister 18 in die zwischenbodenseitige Aufnahme 13 und damit einhergehendem Ausgleichen des Vertikalversatzes in dem Zwischenboden 46 liegt bevorzugt die Deckenunterseite 24 bzw. die deckenunterseitig vorgesehenen Vertikalstege 49 auf dem Blister 18, insbesondere auf der die Abziehhandhabe 17 im freien Endbereich ausbildenden Behälterabdeckung 16 auf. Entsprechend ist der eingelegte Blister 18 in der Deckelverschlussstellung zum einen durch das annähernd formschlüssige Einliegen des Behälterteiles 14 in der Aufnahme 13 und zum weiteren ober- und unterseitig durch die Deckeldecke 20 und der zugeordneten Oberfläche der zwischenbodenseitigen Vertiefung gefasst, wobei weiter eine seitliche Abstützung des Blisters 18 über den Stützabschnitt 15 zum einen durch die Stufe 11 und zum weiteren durch die Randbegrenzung der Vertiefung gegeben ist.

Darüber hinaus stützt sich der Deckel 19 mit den deckelunterseitig vorgesehenen Vertikalstegen 49 auch auf der gegenüber der den Blister 18 aufnehmenden Vertiefung erhabenen, benachbarten Planfläche des Zwischenbodens 46 ab, wobei weiter die Vertikalstege 49 des Deckels 19 zumindest teilweise zugleich Strömungswege begrenzen.

In Längserstreckung der den Blister 18 aufnehmenden Vertiefung in dem Zwischenboden 46 sind beidseitig randseitig der Vertiefung Rastnasen 50 ausgeformt, die in der Behälterteil-Zuordnungsstellung den Stützabschnitt 15 sowie in der Nichtbenutzungsstellung zugleich auch die Behälterabdeckung 16 übergreifen, so dass ein bei geöffnetem Deckel 19 eingelegter Blister 18 zufolge des Übergriffs der Rastnasen 50 in der Zuordnungsstellung gefangen ist. Zufolge der dünnwandigen und gegebenenfalls elastischen Ausgestaltung insbesondere des blisterteilseitigen Stützabschnittes 15 sind die Rastnasen 50 im Zuge des Einsetzens des Blisters 18 wie auch im Zuge der Entnahme zu überwinden.

Darüber hinaus ist oberflächenseitig der zwischenbodenseitigen Vertiefung abzugsseitig der Aufnahme 13 eine Markierung 43 aufgebracht, so insbesondere in Form einer Farbmarkierung. Dieser Markierung 43 ist in Zuordnungsstellung des Blisters 18 in vertikaler Überdeckung zu einem fensterartigen Ausschnitt 51 im Stützabschnitt 15, welcher fensterartige Ausschnitt 51 im Zuge des Abziehens der Behälterabdeckung 16 zur Vorbereitung der Inhalation freigelegt wird.

Der Blister 18 ist derart in den Spender 1 einzulegen, dass die Umschlagkante der Behälterabdeckung 16 unter paralleler Ausrichtung zur Schwenkachse x dem Luftkanal 12 zugewandt angeordnet ist und weiter die frei ausladende Abziehhandhabe 17 in entgegengesetzter Richtung über die Vertiefungsberandung hinaus aus dem Gehäuse 2 ragt, dies unter Durchsetzung einer entsprechend zwischen dem Zwischenboden 46 und in Verschlussstellung verschwenktem Deckel 19 belassenen, schlitzförmigen Abziehöffnung 25. Letztere ist in ihrer quer zur Längserstreckung des Gehäuses 2 betrachteten Breite angepasst an die Breite der Behälterabdeckung 16. Die vertikale Höhe der Abziehöffnung 25 entspricht im Wesentlichen der Materialstärke der Behälterabdeckung 16.

Eine korrekte Ausrichtung des einzulegenden Blisters 18 in dem Spender 1 ist durch eine in Längserstreckung des Spenders 1 bzw. der Vertiefung in dem Zwischenboden 46 vorgesehene außermittige Anordnung der Aufnahme 13 sichergestellt. Der Blister 18 weist entsprechend in Längserstreckung an die außermittige Anordnung der Aufnahme 13 angepasste unterschiedliche Schenkellängen des Stützabschnittes 15 auf, so dass ein Einlegen des Blisters 18 ausschließlich in der vorbestimmten Ausrichtung erreicht werden kann.

Die deckelunterseitig abragenden Vertikalstege 49 bilden in der Deckelschließstellung zusammen mit der zugewandten Oberfläche des Zwischenbodens 46 einen Luftstromkanal 27, welcher mit Bezug auf eine Vertikalprojektion auf den Zwischenboden 46 in Art einer Bogenbahn geführt ist. Der Luftstromkanal 27 führt zunächst als Behälterkanalabschnitt 30 zu dem mit Bezug auf den gehäuseseitigen Luftkanal 12 entfernteren Ende der zwischenbodenseitigen Aufnahme 13 bzw. des in der Aufnahme 13 aufgenommenen Behälterteiles 14. Von diesem Ende ausgehend erstreckt sich der Luftstromkanal 27 unter Ausbildung eines 180°-Krümmungsbereiches 52 bis zu einem zumindest annähernd parallel zur Längsausrichtung der Aufnahme 13 verlaufenden Kanalabschnitt, der weiter unter einseitiger Umfassung der Aufnahme 13 übergeht in einen Direktkanalabschnitt 31.

Anderendig der Aufnahme 13 ist der Luftstromkanal 27 in im Wesentlichen axialer Verlängerung des gehäuseseitigen Luftkanals 12 weiter gebildet, zum unmittelbaren strömungsmäßigen Anschluss an den gehäuseseitigen Luftkanal 12 in der Deckelverschlussstellung. Der Direktkanalabschnitt 31 mündet zusammen mit dem dem Luftkanal 12 zugeordneten Abschnitt des Luftstromkanales 27 in einen Vereinigungsabschnitt 32, welcher in dem dargestellten Ausführungsbeispiel im Bereich der dem Luftstromkanal 27 zugewandten Öffnung des gehäuseseitigen Luftkanals 12 ausgebildet ist.

Der sich insbesondere aus dem Behälterkanalabschnitt 30 und dem Direktkanalabschnitt 31 ausbildende Bogenbahnkanal 53 ist in Richtung auf den Vereinigungsabschnitt 32 hinsichtlich seiner frei durchströmbaren Querschnittsfläche gegenüber der durchschnittlichen Querschnittsfläche des Luftstromkanals 27 verjüngt, so weiter bevorzugt um etwa 40 % der durchschnittlichen Querschnittsfläche. Dieser verjüngte, düsenartige Endabschnitt des Direktkanalabschnittes 31 ist mit dem Bezugszeichen 54 versehen.

In Überdeckung zu der Aufnahme 13 bzw. zu dem in der Aufnahme 13 einliegenden Behälterteil 14 ist deckelunterseitig weiter eine Rippe 55 angeformt. Diese erstreckt sich quergerichtet zwischen zwei, den die Aufnahme 13 übergreifenden Abschnitt des Luftstromkanals 27 begrenzenden Vertikalstegen 49, weist hierbei eine in Deckelschließstellung betrachtete Höhe auf, die so gewählt ist, dass in der Inhalations-Bereitschaftsstellung die nach unten ragende freie Stirnrandkante der Rippe 55 in den nach Abziehen der Behälterabdeckung 16 freigelegten Spalt oberhalb des geöffneten Behälterteils 14 ragt.

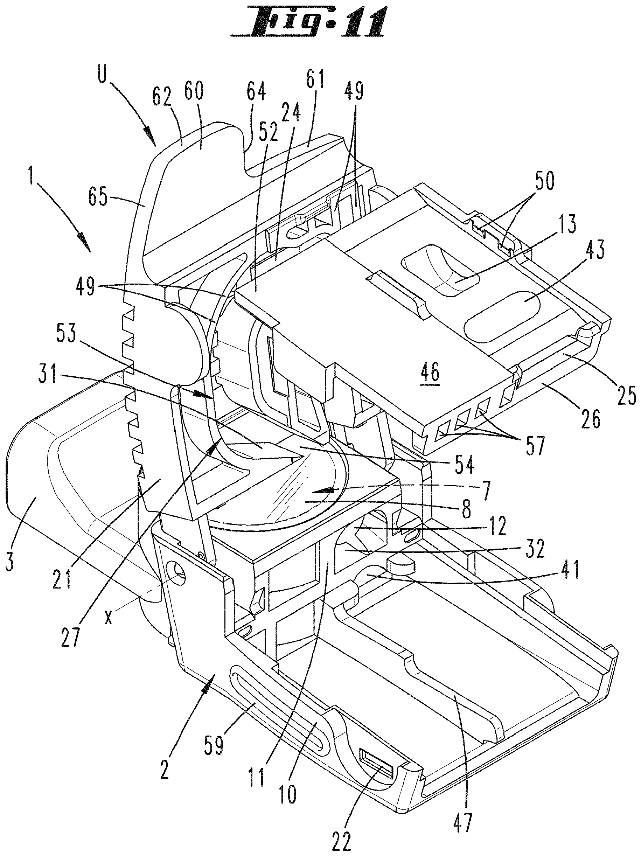

Die Rippe 55 bietet eine Umlenkung des Luftstromkanals 27 in Richtung auf die Aufnahme 13 bzw. auf das in der Aufnahme 13 befindliche Behälterteil 14 sowie eine Umlenkung aus diesem hinaus. Zur zumindest verlustarmen Umlenkung ist die Rippe 55 mit Bezug auf einen Vertikalschnitt gemäß Figur 11 beidseitig mit hohlgewölbten Seitenflächen 56 ausgestattet. Diese sind mit einem bevorzugt an den Querschnittsradius des Luftstromkanals 27 angepassten Radius versehen, wobei weiter die Rippe 55 in ihrem nach unten in Richtung auf die Aufnahme 13 weisenden freien Endbereich eine in Strömungsrichtung betrachtete Breite aufweist, die etwa dem 0,1- bis 0,3-Fachen, bevorzugt etwa dem 0,2-Fachen der in selber Richtung betrachteten Länge der Aufnahme 13 entspricht.

Weiter ist die Rippe 55 mit Bezug auf eine Längserstreckung der Aufnahme 13 bzw. des Behälterteiles 14 mittig und quer zur Längserstreckung ausgerichtet angeordnet.

Der Luftstromkanal 27 ist entsprechend nach Abzug der Behälterabdeckung 16 gemäß Figur 14 zufolge der vorbeschriebenen Anordnung und Ausgestaltung der Rippe 55 im wesentlichen Doppel-S-geführt, dies unter Durchsetzung des Behälterteiles 14.

Des Weiteren sind in der Deckeldecke 20 in Überdeckung zu dem Bogenbahnkanal 53, weiter insbesondere in Überdeckung zu dem Krümmungsbereich 52 Lufteintrittsschlitze 29 vorgesehen, die bevorzugt in der Deckelschließstellung nach oben hin geöffnet sind und weiter auch bevorzugt seitlich die zugeordneten Seitenwand 21 durchsetzend sich öffnen. Es ist so eine einlassgitterartige Abdeckung insbesondere des Krümmungsbereiches 52 erreicht.

Der gehäuseseitige, zum Gehäuseteil 10 hin sich öffnende Luftkanal 12 mündet anderends in einen Umlenkabschnitt 28 der Wirbelkammer 7, weiter in dem oberen Stockwerk 33 der Wirbelkammer 7, welches obere Stockwerk 33 sich im Wesentlichen oberhalb der Trennebene zwischen Zwischenboden 46 und Deckel 19 erstreckt.

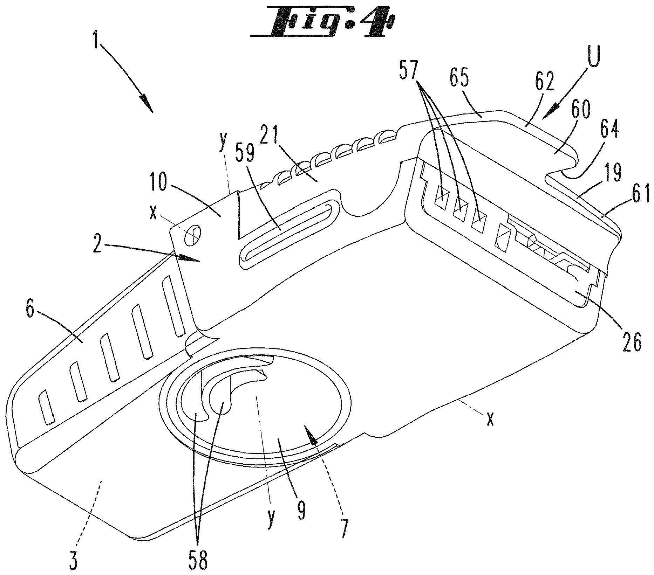

Das obere Stockwerk 33 der Wirbelkammer 7 ist im Wesentlichen ringförmig gestaltet, wozu zentral eine sich über die gesamte Höhe des oberen Stockwerkes 33 erstreckende Ringwandung 34 vorgesehen ist. Ausgehend vom Luftkanaleintritt erstreckt sich der Ringkanal 35 des oberen Stockwerks 33 etwa über einen Winkel von 270° (mit Bezug auf die Darstellung in Figur 4 entgegen Uhrzeigerrichtung) bevor der Ringraum 35 unter Durchsetzen einer den Stockwerksboden 36 öffnenden Durchbrechung 37 übergeht in einen im darunter angeordneten Stockwerk 38 gleichfalls ausgeformten Ringkanal 39. Auch dieser ist radial innen begrenzt durch die im Wesentlichen über die gesamte vertikale Höhe der Wirbelkammer 7 durchlaufende Ringwandung 34.

Unmittelbar in Strömungsrichtung nachgeordnet der bodenseitigen Durchbrechung 37 ist im Bereich des oberen Stockwerks 33 eine den Ringraum in Umströmungsrichtung zum Eintritt des Luftkanals 12 verschließende Trennwand 40 gezogen, die radial von dem zugeordneten Ringwandungsabschnitt ausgeht.

Der Ringkanal 39 des unteren Stockwerks 38 erstreckt sich ausgehend von dem 90°-Krümmungsbereich des oberen Ringkanals 35 über etwa 270° bis zum Anschluss an den Saugkanal 4 des Mundstückes 3.

Die die Stockwerke bzw. die Ringkanäle 35 und 39 verbindende Durchbrechung 37 erstreckt sich über einen Winkelbereich von etwa 180°, dies in Strömungsrichtung betrachtet den Winkelbereich von 90° bis 270° des oberen Ringkanals 35 und den Winkelbereich von 270° bis 90° des unteren Ringkanals 39 aufnehmend. Es ist entsprechend ein Übergang der beiden Ringkanäle über einen Bereich von etwa 180° gegeben.

Es ist so eine im Wesentlichen helixförmige Luftführung in der Wirbelkammer 7 gegeben, dies unter Umlauf der durch die Wirbelkammer gesogenen Luft um insgesamt 360°, dies weiter unter Durchsetzung zweier untereinander angeordneter Ebenen.

In den Ringraum 39 des unteren Stockwerks 38 der Wirbelkammer 7 mündet in vertikaler Überdeckung zu der Mündung des Luftkanals 12 in das obere Stockwerk 33 eine Außenluftöffnung 41, deren Außenluftkanal 42 derart das Gehäuse 2 durchsetzt, dass sich die Außenluftöffnung 41 tangential in den Ringraum 39 hin öffnet. Anderends mündet der Außenluftkanal 42 unter Erstreckung desselben in Längserstreckung des Gehäuses 2 unterseitig der stufenseitigen Öffnung des Luftkanals 12 in den zwischen dem Zwischenboden 46 und dem Gehäuseboden belassenen Zwischenraum 44. Letzterer formt einen Nebenluftkanal 45, der wechselseitig des zwischenbodenseitigen Vertikalsteges 47 ausgebildet ist. Der unterhalb der Aufnahme 13 ausgeformte Bereich des Zwischenraumes 44 ist gehäuserückwärtig durch eine vertikal verlaufende Rückwand 26 des Zwischenbodens 46 verschlossen. In diesem, der Rückwand 26 zugeordneten Endabschnitt ist der den Zwischenraum 44 in Längserstreckung im wesentlichen teilende Vertikalsteg 47 höhenmäßig reduziert, zur strömungsmäßigen Verbindung der nebeneinander ausgebildeten Zwischenbodenbereiche, wobei zugeordnet dem, unterseitig des Bogenbahnkanals 53 ausgeformten Zwischenraumbereich in der Rückwand 26 Außenluft-Eintrittsöffnungen 57 zugeordnet sind. Entsprechend sind diese Außenluft-Eintrittsöffnungen 57 in der Inhalations-Betriebsstellung benachbart der aus dem Gehäuse 2 rückwandseitig herausragenden Behälterabdeckung 16.

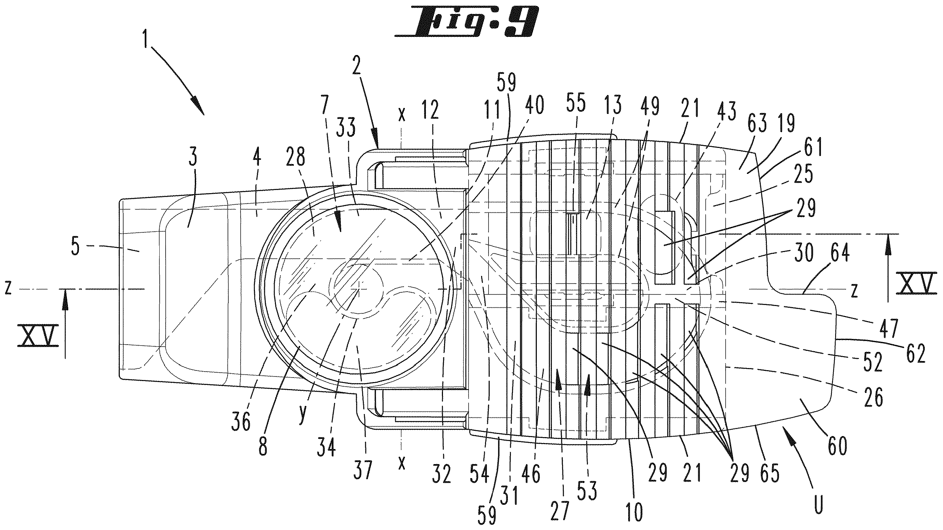

Zum Inhalieren einer Masse M wird zunächst nach Aufschwenken des Deckels 19 in die Offenstellung gemäß Figur 9 der Blister 18 eingelegt derart, dass das Behälterteil 14 in der Aufnahme 13 aufgenommen ist, dies unter Abstützung des blisterseitigen Stützabschnittes 15 auf der zugeordneten, vertieften Oberfläche des Zwischenbodens 46. Die weiter vorgesehenen Rastnasen 50 überfangen hierbei den Stützabschnitt 15 sowie bevorzugt die in Verschlussstellung liegende Behälterabdeckung 16, die weiter zufolge der umschlagseitigen Führung im Endbereich des Blisters 18 insbesondere im Bereich der Rastnasen 50 doppellagig vorliegt. Die frei ausragende Abziehhandhabe 17 steht zur Bedienung frei über das Gehäuseende hinaus.

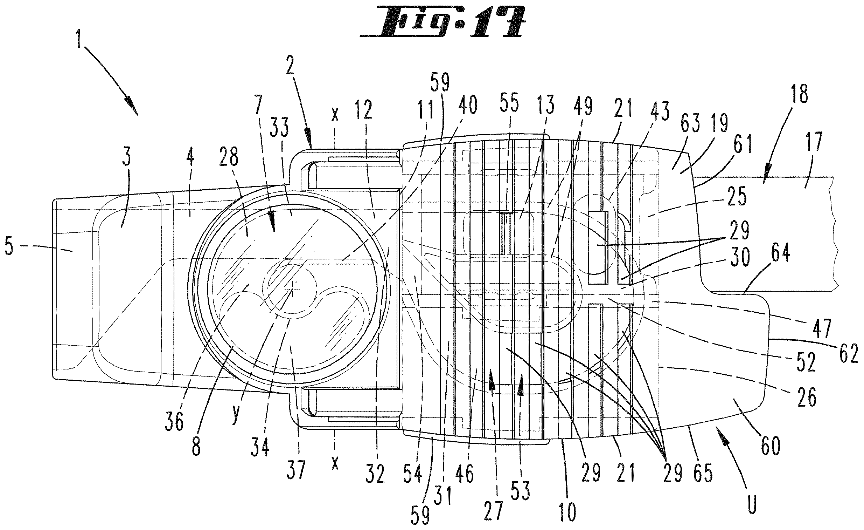

Nach Schließen des Deckels 19 ist der Blister 18 in dem Gehäuse 2 gesichert. Dabei erstreckt sich der Blister 18, insbesondere die Abziehhandhabe 17, seitlich der Verlängerung 60, so insbesondere zugeordnet dem nicht verlängerten Deckelabschnitt 63. Verlängerung 60 und Abziehhandhabe 17 erstrecken sich entsprechend in Nebeneinanderanordnung. Die Abziehhandhabe 17 liegt frei zur Handhabung in dem von der Verlängerung 60 flankierten Freibereich des Deckels 19 (vergleiche Figur 17).

Es erfolgt hiernach das Öffnen des Behälterteiles 14 durch abrollendes Abziehen der Behälterabdeckung 16, wozu die durch die Abziehöffnung 25 hinausragende Abziehhandhabe 17 in Abziehrichtung r gezogen wird. Dieser Abziehvorgang ist zufolge einer transparenten Ausbildung des Deckels 19, insbesondere eine transparente Ausbildung des den Verlagerungsbereich der Behälterabdeckung 16 überdeckenden Abschnitts des Deckels 19 sichtbar. Die auf der Oberfläche des Zwischenbodens 46 vorgesehene Markierung 43 dient dem Benutzer als Hilfsmittel, bis zu welcher Stellung die Behälterabdeckung 16 gezogen werden muss, um eine vollständige Freilegung der in dem Behälterteil 14 bevorrateten Masse M sicherzustellen. Der zunächst von der Behälterabdeckung 60 überdeckte fensterartige Ausschnitt 51 des Stützabschnittes 15 wird im Zuge des abrollenden Abziehens der Behälterabdeckung 16 freigelegt, durch welchen Ausschnitt 51 hiernach die Markierung 43 sichtbar wird. Entsprechend ist für den Benutzer eine Orientierung zum ordnungsgemäßen Gebrauch des Spenders 1 gegeben.

Nach Abziehen der Behälterabdeckung vom Behälterteil 14 liegt die Masse M im Strömungsweg frei. Das Behälterteil 14 ist bedingt durch die fehlende Abdeckung nunmehr Teil des Strömungsweges, entsprechend strömungsmäßig einerends angeschlossen an den zum Vereinigungsabschnitt 32 und weiter zum Luftkanal 12 führenden Abschnitt des Luftstromkanals 27 und anderends an den bogenbahnkanalartigen Abschnitt des Luftstromkanals 27, welcher endseitig wiederum in den Vereinigungsabschnitt 32 mündet.

Der Spender 1 wird zur Inhalation flötenartig, bevorzugt mit Daumen und Zeigefinger gehalten. Durch Ansaugen über das Mundstück 3 tritt Luft durch die Einlassschlitze 29 in den Bogenbahnkanal 53 des Luftstromkanals 27 ein, wonach sich die eintretende Luft aufteilt zur Durchsetzung des unmittelbar zum Vereinigungsabschnitt 32 führenden Direktkanalabschnittes 31 und zur Durchsetzung des unmittelbar zum Behälterteil 14 führenden Behälterkanalabschnittes 30. Über diesen Behälterkanalabschnitt 30 wird die in dem Behälterteil 14 bevorratete pulverförmige Masse M ausgetragen, wonach sich der massenversetzte Luftanteil b in dem Vereinigungsabschnitt 32 mit dem Luftanteil a durch den Direktkanalabschnitt 31 vereinigt. Zufolge der zum Vereinigungsabschnitt 32 hin sich verjüngenden Querschnittsausgestaltung des Direktkanalabschnittes 31 wird auf den ebenfalls in den Vereinigungsabschnitt 32 einmündenden Abschnitt des Luftstromkanals 27 eine Sogwirkung erzielt, die das Ausräumen des Behälterteiles 14 durch die über den Behälterkanalabschnitt 30 eingebrachte Blasluft unterstützt. Unter Durchsetzung der spiralförmigen, doppelstöckigen Wirbelkammer 7 erfolgt eine gleichmäßige Verteilung der Masse M im Luftstrom. Vor Austritt aus der Wirbelkammer 7 in den Saugkanal 4 des Mundstückes 3 tritt weitere Außenluft c über die Außenluftöffnung 41 bei. Die hierbei nebenluftartig angesogene Außenluft c tritt durch den Außenluftkanal 42 in die Wirbelkammer 7, wobei die in das untere Stockwerk 38 einmündende Außenluftöffnung 41 in Richtung auf die gegenüberliegende Öffnung zum Saugkanal 4 gerichtet ist. Der Außenluftstrom c vereinigt sich in dem unteren Stockwerk 33 der Wirbelkammer 7 mit dem massenbehafteten Luftstrom a, b unmittelbar vor Übergang in den Saugkanal 4.

Durch die dachartige Verlängerung 60 des Deckels 19 (vergleiche hierzu auch die Schnittdarstellung in Figur 6) sind die Außenluft-Eintrittsöffnungen 57, durch welche die Außenluft c angesaugt wird, in einer geschützten Lage, so dass diese bei einem üblichen Inhalationsvorgang nicht, beispielsweise durch einen Finger, versehentlich verdeckt werden können.

Im Zuge des Inhaliervorganges verschließt die freie Behälterabdeckungs-Fahne zumindest größtenteils die Abziehöffnung 25, womit einem Ansaugen von Nebenluft bevorzugt entgegengewirkt ist.

Zufolge der beschriebenen Ausgestaltung erfolgt die Ansaugung bzw. die Luftführung innerhalb des Gehäuses 2 unter Umgehung der zur Inhalation in die Abziehstellung verlagerten Behälterabdeckung 16. Der die Masse M aus dem Behälterteil 14 ausräumende Saugluftstrom ist irritationsfrei geführt. Zufolge der bogenbahnkanalartigen Führung des Luftstromkanals 27, weiter insbesondere auch zufolge der düsenartigen Verjüngung des Direktkanalabschnittes 31 in Richtung auf den Vereinigungsabschnitt 32 ist die Luftführung insbesondere des massenbehafteten Luftstromes optimiert. Der Spender 1 ist geeignet zur Inhalation unterschiedlicher Massen M, insbesondere sich hinsichtlich der Körnung und/ oder Mischung unterscheidende Massen.

**Liste der Bezugszeichen**

| | | | |
|---|---|---|---|
| 1 | Spender | 29 | Lufteintrittsschlitz |
| 2 | Gehäuse | 30 | Behälterkanalabschnitt |
| 3 | Mundstück | 31 | Direktkanalabschnitt |
| 4 | Saugkanal | 32 | Vereinigungsabschnitt |
| 5 | Saugmund | 33 | Stockwerk |
| 6 | Verschlusskappe | 34 | Ringwandung |
| 7 | Wirbelkammer | 35 | Ringkanal |
| 8 | Kammerdecke | 36 | Stockwerksboden |
| 9 | Kammerbodeen | 37 | Durchbrechung |
| 10 | Gehäuseteil | 38 | Stockwerk |
| 11 | Stufe | 39 | Ringkanal |
| 12 | Luftkanal | 40 | Trennwand |
| 13 | Aufnahme | 41 | Außenluftöffnung |
| 14 | Behälterteil | 42 | Außenluftkanal |
| 15 | Stützabschnitt | 43 | Markierung |
| 16 | Behälterabdeckung | 44 | Zwischenraum |
| 17 | Abziehhandhabe | 45 | Nebenluftkanal |
| 18 | Blister | 46 | Zwischenboden |
| 19 | Deckel | 47 | Vertikalsteg |
| 20 | Decke | 48 | --- |
| 21 | Seitenwand | 49 | Vertikalsteg |
| 22 | Rastvertiefung | 50 | Rastnase |
| 23 | Rastvorsprung | 51 | Ausschnitt |
| 24 | Deckenunterseite | 52 | Krümmungsbereich |
| 25 | Abziehöffnung | 53 | Bogenbahnkanal |
| 26 | Rückwand | 54 | verjüngter Abschnitt |
| 27 | Luftstromkanal | 55 | Rippe |
| 28 | Umlenkabschnitt | 56 | Hohlfläche |
| 57 | Außenluft-Eintrittsöffnung | a | Luftstrom |
| 58 | Ausnehmung | b | Luftstrom |
| 59 | Vorsprungausbildung | c | Luftstrom |
| 60 | Verlängerung | d | Länge |
| 61 | Randfläche | d' | Länge |
| 62 | Stirnrandfläche | e | Breite |
| 63 | Deckelabschnitt | f | Breite |
| 64 | Seitenrandfläche | g | Versatzmaß |
| 65 | Seitenrandfläche | h | Hilfslinie |
| 66 | Schnittpunkt | j | Hilfslinie |
| 67 | Schnittpunkt | k | Hilfslinie |
| | | m | Radius |
| | | n | Radius |
| | | p | Ausmaß |
| | | q | Länge |
| | | r | Abziehrichtung |
| | | x | Schwenkachse |
| | | y | Wirbelkammerachse |
| | | z | Längsmittelachse |
| | | L | Längserstreckung |
| | | M | Masse |
| | | U | Untergreifvorsprung |

## Patentansprüche

1. Spender (1) für in einer gesonderten Verpackung enthaltene, pulverförmige Massen (M), insbesondere in einer Blisterverpackung (18) enthaltene Massen (M), wobei die Verpackung ein Behälterteil (14) mit abziehbarer Behälterabdeckung (16) aufweist, wobei weiter ein eingesaugter Luftstrom nach Entfernen der Behälterabdeckung (16) vom Behälterteil (14) zum überwiegenden Teil durch das Behälterteil (14) gesaugt wird, derart, dass zwei durch Schlitze (29) eines um eine Schwenkachse (x) schwenkbaren Deckels (19) eintretende Luftströme (a, b) das Behälterteil (15) von seinen beiden Enden her entleeren und nach Vereinigung in eine, einem Mundstück (3) vorgeschaltete Wirbelkammer (7) gelangen, wobei darüber hinaus in einer radialen Erstreckung zu der Schwenkachse (x) in einem von dem Deckel (19) abgedeckten Gehäuseteil (10) Außenluft- Eintrittsöffnungen (57) für einen dritten Luftstrom (c) ausgebildet sind, wobei der Deckel (19) in einem, den Außenluft- Eintrittsöffnungen (57) für den dritten Luftstrom (c), zugeordneten Teilbereich über eine Breite (f), welche einer Hälfte oder weniger der Breite (e) des Deckels (19) entspricht, mit einer bezüglich der Schwenkachse (x) radialen Verlängerung (60) ausgebildet ist, **dadurch gekennzeichnet, dass** die Verlängerung (60) zugleich einen Untergreifvorsprung (U) zum Öffnen des Deckels (19) bildet und über einen benachbarten, nicht verlängerten Deckelabschnitt (63) hinweg frei ausragt und das Ausmaß (p) der Verlängerung (60) im Hinblick auf den nicht verlängerten Deckelabschnitt (63) einem Fünftel oder mehr der Breite (e) des Deckels (19) entspricht.

2. Spender nach Anspruch 1, **dadurch gekennzeichnet, dass** die Verlängerung (60) einstückig und/oder materialeinheitlich mit dem Deckel (19) ausgebildet ist.

3. Spender nach einem der vorhergehenden Ansprüche, **dadurch gekennzeichnet, dass** die deckelseitige Verlängerung (60) die in dem Gehäuseteil (10) vorgesehenen Außenluft-Eintrittsöffnungen (57) in der Deckelverschlussstellung vordachartig überfängt.

4. Spender nach einem der vorhergehenden Ansprüche, **dadurch gekennzeichnet, dass** der benachbart zu der Verlängerung (60) ausgebildete, eine Randfläche (61) aufweisender Deckelabschnitt (63) ähnlich der Verlängerung (60) über die Rückwand (26) hinausragt, mit einem vergleichsweise geringen Längenmaß (d'), das dem 0,2 bis 0,5-Fachen des Längenmaßes (d) entspricht, mit dem die Verlängerung (60) über die Rückwand (26) hinausragt, welche Länge (d) dem 0,25 bis 0,4-Fachen der Deckellänge (q), abgetragen von der Schwenkachse (x) bis zu einer freien Stirnrandfläche (62) der Verlängerung (60), entspricht.

## Claims

1. Dispenser (1) for pulverulent masses (M) contained in a separate package, in particular masses (M) contained in a blister package (18), the package having a container part (14) with a removable container cover (16), wherein furthermore a sucked-in air stream is sucked for the most part through the container part (14) after removal of the container cover (16) from the container part (14), in such a way, in such a way that two air streams (a, b) entering through slits (29) of a cover (19) pivotable about a pivot axis (x) empty the container part (15) from its two ends and, after merging, pass into a swirl chamber (7) connected upstream of a mouthpiece (3), wherein, in addition, external air inlet openings (57) for a third air stream (c) are formed in a housing part (10) covered by the cover (19) in a radial extension to the pivot axis (x), wherein the cover (19), in a partial region associated with the external air inlet openings (57) for the third air flow (c), is formed over a width (f) which corresponds to half or less of the width (e) of the cover (19), with a radial extension (60) with respect to the pivot axis (x), **characterized in that** the extension (60) at the same time forms an undergripping projection (U) for opening the lid (19) and projects freely beyond an adjacent, non-extended lid section (63), and the extent (p) of the extension (60) with respect to the non-extended lid section (63) corresponds to one fifth or more of the width (e) of the lid (19).

2. Dispenser according to claim 1, **characterized in that** the extension (60) is formed integrally and/or of the same material as the lid (19).

3. Dispenser according to one of the preceding claims, **characterized in that** the extension (60) on the lid side overlaps the outside air inlet openings (57) provided in the housing part (10) in the manner of a porch in the lid closure position.

4. Dispenser according to one of the preceding claims, **characterized in that** the cover portion (63) formed adjacent to the extension (60) and having an edge surface (61) projects beyond the rear wall (26) in a manner similar to the extension (60), with a comparatively small length dimension (d'), which corresponds to 0.2 to 0.5 times the length dimension (d) with which the extension (60) projects beyond the rear wall (26), which length (d) corresponds to 0.25 to 0.4 times the cover length (q), removed from the pivot axis (x) to a free end edge surface (62) of the extension (60).

## Revendications

1. Distributeur (1) pour des masses pulvérulentes (M) contenues dans un emballage séparé, en particulier des masses (M) contenues dans un emballage blister (18), l'emballage présentant une partie de récipient (14) avec un couvercle de récipient (16) pouvant être retiré, un flux d'air aspiré étant en outre aspiré en majeure partie à travers la partie de récipient (14) après le retrait du couvercle de récipient (16) de la partie de récipient (14), de telle manière, que deux flux d'air (a, b) entrant par des fentes (29) d'un couvercle (19) pouvant pivoter autour d'un axe de pivotement (x) vident la partie de récipient (15) par ses deux extrémités et parviennent, après avoir été réunis, dans une chambre de tourbillonnement (7) placée en amont d'une embouchure (3), des ouvertures d'entrée d'air extérieur (57) pour un troisième flux d'air (c) étant en outre formées dans une extension radiale par rapport à l'axe de pivotement (x) dans une partie de boîtier (10) recouverte par le couvercle (19), le couvercle (19) étant formé dans une zone partielle associée aux ouvertures d'entrée d'air extérieur (57) pour le troisième flux d'air (c), sur une largeur (f) qui correspond à la moitié ou moins de la largeur (e) du couvercle (19), avec un prolongement (60) radial par rapport à l'axe de pivotement (x), **caractérisé en ce que** le prolongement (60) est formé dans la zone partielle du couvercle (19), **en ce que** le prolongement (60) forme en même temps une saillie de préhension inférieure (U) pour l'ouverture du couvercle (19) et fait saillie librement au-delà d'une section de couvercle (63) voisine, non prolongée et l'étendue (p) du prolongement (60) correspond, par rapport à la section de couvercle (63) non prolongée, à un cinquième ou plus de la largeur (e) du couvercle (19).

2. Distributeur selon la revendication 1, **caractérisé en ce que** le prolongement (60) est formé d'une seule pièce et/ou d'un seul matériau avec le couvercle (19).

3. Distributeur selon l'une des revendications précédentes, **caractérisé en ce que** le prolongement (60) côté couvercle recouvre à la manière d'un avanttoit les ouvertures d'entrée d'air extérieur (57) prévues dans la partie de boîtier (10) dans la position de fermeture du couvercle.

4. Distributeur selon l'une des revendications précédentes, **caractérisé en ce que** la section de couvercle (63) formée au voisinage du prolongement (60) et présentant une surface de bord (61) dépasse de la paroi arrière (26) de manière similaire au prolongement (60), avec une mesure de longueur (d') comparativement faible, qui correspond à 0,2 à 0,5 fois la mesure de longueur (d) avec laquelle le prolongement (60) dépasse de la paroi arrière (26), laquelle longueur (d) correspond à 0,25 à 0,4 fois la longueur du couvercle (q), décomptée depuis l'axe de pivotement (x) jusqu'à une surface de bord frontal libre (62) du prolongement (60).
